# EUROPEAN PATENT SPECIFICATION

(11) **EP 0 792 174 B1**
(45) Date of publication and mention of the grant of the patent: **03.03.1999**
(21) Application number: 95936667.5
(22) Date of filing: 13.11.1995
(51) Int. Cl.: A61M 5/30

(54) **MEDICAL GLASS CONTAINER SUITABLE FOR USE IN NEEDLELESS INJECTORS**
MEDIZINISCHER GLASBEHÄLTER GEEIGNET ZUM GEBRAUCH IN NADELLOSEN SPRITZENVORRICHTUNGEN
FLACON EN VERRE MEDICAL POUR INJECTEURS PNEUMATIQUES SOUS-CUTANES

(30) Priority: 19.11.1994 GB 9423505
(43) Date of publication of application: 03.09.1997
(73) Proprietor: WESTON MEDICAL LIMITED, Stradbroke, Eye, Suffolk IP21 5JG (GB)
(72) Inventor: WESTON, Terence, Edward, Eye Suffolk IP21 5NG (GB); LICENCE, Anthony, Philip, Suffolk IP12 4HR (GB)
(74) Representative: Boon, Graham Anthony
(86) International application number: GB9502649
(87) International publication number: WO9615821

(56) References cited:
- EP-A- 0 584 531
- US-A- 3 650 084
- US-A- 3 688 765
- US-A- 3 782 380

## Description

The invention relates to a glass container, adapted to withstand a high internal pressure, for use as a capsule in a needleless injector.

International Specification No. WO 95/03844 discloses a needleless injector for delivering a liquid medicament into the skin. During the injection, very high pressures of short duration are generated. A preferred embodiment of that invention requires a liquid medicament to be stored in the dispenser in a glass container, preferably a cylindrical glass container, which also serves as the pressure vessel. In order to withstand these high pressures, the container may be provided with thick walls. However, these make it more difficult and costly to make. These size and cost disadvantages partly offset the potential benefits of needleless injectors compared with conventional hypodermic syringes. It is an object of a preferred embodiment of the present invention to provide a glass container which can be used in such a needleless injector, without it having to be provided with such thick walls.

US-A-3688765 describes a glass container adapted for use as a needleless injector and comprising a hollow glass body.

The invention employs the principle that glass is able to withstand higher forces if the surfaces are maintained in a state of compressive stress. The accepted theories on glass fracture propose that breakage occurs through the rapid growth of microcracks on the glass surface when the glass is stressed, in addition to fractures originating from gross surface defects. By holding the surfaces in compressive stress, this stress must be overcome before crack growth can occur.

According to the present invention there is provided a container of the type disclosed in US-A-3688765, characterized by compression means for exerting a compressive force on the hollow glass body, whereby the container is adapted to withstand a high internal pressure.

In one embodiment thereof the compression means is a tight fitting sleeve assembled onto the capsule body so as to exert a compressive force on a preferably cylindrical wall thereof.

It has been found that a borosilicate glass cylinder having a bore of 5mm diameter and a wall thickness of 2.19mm will sometimes break when subjected to internal pressures of 80 bars and transient pressures of 4000 bars. Furthermore, the cylinders are unable to withstand more than two applications of such pressures. A polycarbonate sleeve having a nominal interference fit of 0.05mm enables the cylinders to withstand at least 20 applications of such pressures without damage.

An embodiment of the invention is shown in the single figure of drawing, which is a diagrammatic longitudinal section.

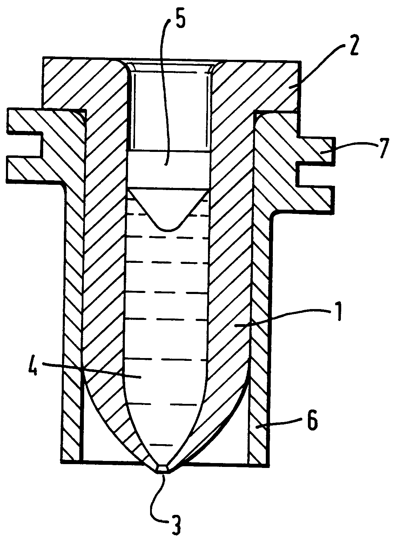

Figure 1 shows a glass needleless injection capsule 1 having a main body portion defined by a wall which is cylindrical both internally and externally, a flange 2 at one end thereof, and a tapered portion at the other end terminating in a discharge orifice 3. The capsule 1 contains injectate 4 and a free, slidable piston 5. A sleeve 6 is an interference fit onto the cylindrical wall of the capsule 1. A thread 7 on the sleeve 6 enables the assembly to be screwed into and retained by an injector actuator (not shown). There are many alternative methods of securing the assembly to the actuator, such as snap fitting, or ultrasonic welding, adhesives, or metal clips, to name a few examples.

In use, the discharge orifice 3 is placed on the subject's skin, and the free piston 5 is struck by the ram of the actuator. This blow is transferred to the injectate 4 causing a rapid rise in pressure. This causes a corresponding tensile stress in the walls of the capsule 1, tending to burst it. This is prevented by the compressive stress induced by the tight-fitting sleeve 6 which partly or fully resists the tensile stress resulting from the hydraulic impact.

Thus it may be seen that the invention prevents the breakage of glass capsules when subjected to high pressure. It also protects the glass surface from damage during storage.

The sleeve 6 is preferably made from a transparent plastic, such as a polycarbonate, polyethertetrapthalate, polystyrene, or cellulose ester (e.g. cellulose acetate-propionate), which enables the contents to be examined. The strength of the glass may be further improved by ion stuffing, thermal pre-stressing, or etching, and the present specification allows for all combinations of materials and processes which will achieve the object of the invention.

## Claims

1. A glass container (1) adapted for use as a needleless injector capsule and comprising a hollow glass body, characterized by compression means (6) for exerting a compressive force on the hollow glass body, whereby the container is adapted to withstand a high internal pressure.

2. A container according to claim 1, wherein the hollow glass body is generally cylindrical over at least a portion thereof.

3. A container according to claim 1 or 2, comprising a main body portion defined by an internally cylindrical wall and having a flange at one end thereof, and a tapered portion at the other end terminating in a discharge orifice (3).

4. A container according to claim 3, wherein the wall of the main body portion is also externally cylindrical.

5. A glass container according to any preceding claim, wherein the compression means is a sleeve (6) which fits tightly around the hollow glass body.

6. A container according to claim 5, wherein the sleeve (6) is of a plastics material.

7. A container according to claim 6, wherein the plastics material is transparent.

8. A container according to claim 7, wherein the plastics material is selected from the group consisting of polycarbonates, polyethertetraphthalates, polystyrenes and cellulose esters.

9. A container according to claim 8, wherein the plastics material is cellulose acetate-propionate.

10. A container according to any preceding claim, having a free, slidable piston (5) within the hollow glass body.

## Patentansprüche

1. Glasbehälter (1) für den Gebrauch als nadelllose Injektionskapsel, welcher einen hohlen Glaskörper aufweist, dadurch gekennzeichnet, daß Kompressionsmittel (6) vorgesehen sind, um eine Kompressionskraft auf den hohlen Glaskörper auszuüben, um auf diese Weise den Behalter in die Lage zu versetzen, einen hohen Innendruck auszuhalten.

2. Glasbehälter nach Anspruch 1, dadurch gekennzeichnet, daß der hohle Glaskörper mindestens in einem Teil dieses Glaskörpers eine zylindrische Form hat.

3. Glasbehälter nach einem der Ansprüche 1 oder 2, dadurch gekennzeichnet, daß ein Hauptkörper vorgesehen ist, welcher durch eine zylindrische Innenwand gebildet wird, und an einem seiner Enden einen Flansch und an seinem anderen Ende einen abgestuften Abschnitt aufweist, welcher in eine Abgabeöffnung (3) mündet.

4. Glasbehälter nach Anspruch 3, dadurch gekennzeichnet, daß die Wand des Hauptkörpers auch an seiner Außenseite zylindrisch gestaltet ist.

5. Glasbehälter nach einem der vorausgegangenen Ansprüche, dadurch gekennzeichnet, daß die Kompressionsmittel aus einer Manschette (6) bestehen, welche im festen Sitz um den hohlen Glaskörper angeordnet ist.

6. Glasbehälter nach Anspruch 5, dadurch gekennzeichnet, daß die Manschette (6) aus einem Kunststoff hergestellt ist.

7. Glasbehälter nach Anspruch 6, dadurch gekennzeichnet, daß der Kunststoff transparent ist.

8. Glasbehälter nach Anspruch 7, dadurch gekennzeichnet, daß der Kunststoff aus einer Gruppe ausgewählt ist, welche aus Polykarbonat, Polyethertetraphtalat, Polystyrol und Zelluloseester besteht.

9. Glasbehälter nach Anspruch 8, dadurch gekennzeichnet, daß der Kunststoff ein Zelluloseazetatpropionat ist.

10. Glasbehälter nach einem der vorausgegangenen Ansprüche, dadurch gekennzeichnet, daß er in seinem hohlen Glaskörper einen frei gleitenden Kolben (5) enthält.

## Revendications

1. Flacon en verre (1) conçu pour être utilisé comme capsule d'injecteur sans aiguille et comprenant un corps en verre creux, caractérisé par des moyens de compression (6) permettant d'exercer une force de compression sur le corps en verre creux, si bien que le flacon est conçu pour résister à une forte pression interne.

2. Flacon selon la revendication 1, dans lequel le corps en verre creux est globalement cylindrique sur au moins une des ses parties.

3. Flacon selon la revendication 1 ou 2, comprenant une partie de corps principal définie par une paroi cylindrique à l'intérieur et présentant une bride à une de ses extrémités, et une partie conique à l'autre extrémité se terminant par un orifice d'évacuation (3).

4. Flacon selon la revendication 3, dans lequel la paroi de la partie de corps principal est également cylindrique à l'extérieur.

5. Flacon en verre selon l'une quelconque des revendications précédentes, dans lequel les moyens de compression sont un manchon (6) qui s'adapte solidement autour du corps en verre creux.

6. Flacon selon la revendication 5, dans lequel le manchon (6) est en matériau plastique.

7. Flacon selon la revendication 6, dans lequel le matériau plastique est transparent.

8. Flacon selon la revendication 7, dans lequel le matériau plastique est choisi dans le groupe composé des polycarbonates, des polyéthertétraphtalates, des polystyrènes et des esters de cellulose.

9. Flacon selon la revendication 8, dans lequel le matériau plastique est de l'acétate-propionate de cellulose.

10. Flacon selon l'une quelconque des revendications précédentes, présentant un piston (5) libre pouvant coulisser à l'intérieur du corps en verre creux.
